# EUROPÄISCHE PATENTSCHRIFT

(11) **EP 1 836 204 B1**
(45) Veröffentlichungstag und Bekanntmachung des Hinweises auf die Patenterteilung: **21.10.2009**
(21) Anmeldenummer: 05822589.7
(22) Anmeldetag: 08.12.2005
(51) Int. Cl.: C07D 471/08, C07B 59/00, A61K 33/34, A61K 51/04, A61K 103/00

(54) **RADIOAKTIVE METALLKOMPLEXE AUF DER BASIS VON BISPIDIN UND DESSEN DERIVATE**
RADIOACTIVE METAL COMPLEXES BASED ON BISPIDINE AND DERIVATIVES THEREOF
COMPLEXES MÉTALLIQUES RADIOACTIFS À BASE DE BISPIDINE ET DE SES DÉRIVÉS UTILISÉS EN TANT QU'AGENTS CHÉLATEURS, ET UTILISATION DE CES COMPLEXES MÉTALLIQUES À DES FINS DE DIAGNOSTIC ET DE THÉRAPIE EN MÉDECINE NUCLÉAIRE

(30) Priorität: 24.12.2004 DE 102004062568
(43) Veröffentlichungstag der Anmeldung: 26.09.2007
(73) Patentinhaber: Forschungszentrum Dresden - Rossendorf e.V., 01328 Dresden (DE)
(72) Erfinder: COMBA, Peter, 69527 Wiesenbach (DE); JURAN, Stefanie, 01979 Kostebrau (DE); KERSCHER, Marion, 68535 Edingen (DE); PIETZSCH, Hans-Jürgen, 01809 Heidenau (DE); SPIES, Hartmut, 01217 Dresden (DE); STEPHAN, Holger, 01188 Dresden (DE)
(86) Internationale Anmeldenummer: PCT/EP2005/013171
(87) Internationale Veröffentlichungsnummer: WO 2006/072336

(56) Entgegenhaltungen:
- BLOWER P J ET AL: "Copper Radionuclides and Radiopharmaceuticals in Nuclear Medicine" NUCLEAR MEDICINE AND BIOLOGY, ELSEVIER, NY, US, Bd. 23, Nr. 8, November 1996 (1996-11), Seiten 957-980, XP004073004 ISSN: 0969-8051 in der Anmeldung erwähnt

## Beschreibung

Die Erfindung betrifft spezifische radioaktive Metallkomplexe auf der Basis von Bispidin und dessen Derivate als Chelatbildner und deren Verwendung für die nuklearmedizinische Diagnostik und Therapie sowie ein Verfahren zur Herstellung derartiger radioaktiver Metallkomplexe.

Die Nuklearmedizin als ein wichtiges Teilgebiet der modernen Medizin nutzt offene radioaktive Substanzen, sog. Radiopharmaka, zu diagnostischen und therapeutischen Zwecken. Die überwiegende Anzahl von Radionukliden mit diagnostisch und therapeutisch nutzbaren Strahlungseigenschaften sind ihrer chemischen Natur nach Metalle und daher spielen Arbeiten zur Entwicklung von Metalloradiopharmaka eine wichtige Rolle in der modernen Radiopharmakaforschung (C. J. Anderson, M. J. Welch: "Radiometal-labeled agents (non-technetium) for diagnostic imaging". Chem. Rev. 99 (1999) 2219-2234; W. A. Volkert, T. J. Hoffmann: "Therapeutic radiopharmaceuticals". Chem. Rev. 99 (1999) 2269-2292).

Ein wesentlicher begrenzender Faktor bei der Entwicklung von Metalloradiopharmaka im allgemeinen und im besonderen auch für Kupferradiopharmaka ist der Mangel an geeigneten Komplexbildnern, mit denen das radioaktive Metall stabil an Biomoleküle angekoppelt werden kann. Insbesondere ist die Stabilität vieler Metallchelate hinsichtlich Hydrolyse, die unter Freisetzung des radioaktiven Metalls verläuft, unzureichend.

Kupferisotope sind sowohl für eine diagnostische als auch therapeutische Anwendung in der Endoradionuklidtherapie von Interesse. Das betrifft die Positronenemitter ⁶⁰Cu, ⁶¹Cu, ⁶²Cu und ⁶⁴Cu für die Positronen-Emissions-Tomographie (PET) und ⁶⁴Cu sowie das β⁻-emittierende ⁶⁷Cu (Eₘₐₓ = 0.576 MeV) als attraktive Radioisotope für Radiotherapie und Radioimmuntherapie (W. A. Volkert, T. J. Hoffmann a.a.O.; J. S. Lewis, M. J. Welch: "Copper Chemistry related to Radiopharmaceutical Production". In M.Nicolini, U.Mazzi (Hrsg.) Proc. of 6. Intern.Symp. on Technetium in Chemistry and Nuclear Medicine, (Bressanone, Sept. 2002) p. 23 - 33).

Die Nuklideigenschaften von ⁶⁴Cu erlauben dessen Verwendung für PET-Imaging und Radiotherapie. Daher gestatten mit ⁶⁴Cu-markierte Radiopharmaka eine Radiotherapie mit gleichzeitigem Monitoring der Verteilung und Biokinetik durch PET-Imaging. Das Nuklid ist in guten Ausbeuten und hoher spezifischer Aktivität in kleinen Zyklotronen zu erhalten und steht daher zu niedrigen Kosten für die Routinepräparation von Radiopharmaka für die Behandlung beispielsweise von Tumorerkrankungen zur Verfügung.

Ungeachtet dessen gibt es bisher nur wenige auf radioaktiven Kupferisotopen basierende Radiopharmaka. Beispiele sind einmal ⁶⁰Cu-ATSM als Hypoxie-Marker bzw. ⁶⁴Cu-ATSM und ⁶⁴Cu-PTSM, die als potentielle Agentien zur Tumortherapie diskutiert werden. Weitere Stoffklassen sind kupfermarkierte Peptide und Antikörper, in denen das radioaktive Kupfer über einen bifunktionellen Chelator an das Siomolekül gebunden ist.

Wie bei Metalloradiopharmaka bereits ausgeführt, erfordert auch die Entwicklung von Kupferradiopharmaka geeignete Komplexbildner, mit denen das radioaktive Metall hydrolytisch und radiochemisch stabil gebunden werden kann. Die gegenwärtig ungenügende Verfügbarkeit solcher Komplexbildner ist ein gravierender begrenzender Faktor in der Entwicklung von Kupferradiopharmaka. Aus Arbeiten zur Entwicklung kupfermarkierter Antikörper haben sich cyclische Tetraaza-Verbindungen als die am häufigsten verwendeten Komplexbildner herausgestellt (P. J. Blower, J. S. Lewis, J. Zweit: "Copper Radionuclides and Radiopharmaceuticals in Nuclear Medicine". Nucl. Med. Biol. 23 (1996) 957-980; D. Parker: "Imaging and Targeting" in Comprehensive Supramolecular Chemistry, vol. 10 "Supramolecular Technology", J. L Atwood, J. E. D. Davis, D. P. Mac Nicol, F. Vögtle, J.-M. Lehn (Hrsg.), Pergamon 1996, 487-536.) Jedoch erwiesen sich auch diese Verbindungen als insbesondere für eine therapeutische Anwendung ungeeignet, da sie zu unspezifisch wirken.

Als Grundvoraussetzung für Metallkomplexe, die mit Aussicht auf Erfolg als Radioparmaka einsetzbar sind, werden sehr hohe Komplexstabilitäten und metabolische Stabilität hinsichtlich des Chelats gefordert. Da die Wahl des Komplexbildners Biokinetik, Verteilung und Metabolismus der radioaktiven Verbindung dramatisch beeinflusst, sollte der Komplexbildner darüber hinaus strukturell variierbar sein, um Bioverteilungsmuster der Metall-Biomolekül-Konjugate optimieren zu können.

Somit liegt der vorliegenden Erfindung die Aufgabe zugrunde, radioaktive Metallkomplexe bereitzustellen, in denen die radioaktive Metalle mit hoher Stabilität gebunden sind und die nach Applikation in den Körper metabolisch stabil sind.

Diese Aufgabe wird durch die in den Ansprüchen gekennzeichneten Ausführungsformen gelöst.

Insbesondere werden radioaktive Metallkomplexe auf Basis von Bispidin und dessen Derivate (Bispidin = 3,7-Diazabicyclo[3.3.1]nonan) als Chelatbildner mit der folgenden allgemeinen Formel (I) bereitgestellt, 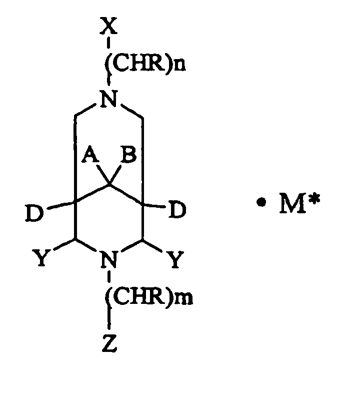 worin
A und B unabhängig voneinander für H, -OR¹, -SR¹, -NHR¹, -OC(O)R¹, -NHC(O)R¹ oder einen geradkettigen oder verzweigtkettigen (C₁₋₈) Alkylrest stehen oder A und B zusammen für =O oder =S stehen, wobei R¹ Wasserstoff, einen geradkettigen oder verzweigtkettigen (C₁₋₈) Alkylrest, einen substituierten oder unsubstituierten (C₆₋₁₀) Arylrest, insbesondere Phenyl, darstellt,
D jeweils eine Carboxylgruppe oder ein davon abgeleitetes Derivat, ausgewählt aus Estern, Amiden und Peptiden, ist,
X, Y und Z unabhängig voneinander einen unsubstituierten oder substituierten Heterocyclus, ausgewählt aus Pyridin, Pyrimidin, Pyrazin, Thiophen, Furan, Pyrazol, Imidazol, Thiazol, Chinolin, Chinazolin und Chinoxalin, darstellen, wobei die Substituenten aus geradkettigen oder verzweigtkettigen (C₁₋₈) Alkylresten, Halogen, geradkettigen oder verzweigtkettigen (C₁₋₈) Alkoxyresten, Sulfonat, Carboxylat, Nitro, Cyan, Hydroxy, Benzyl oder Phenyl ausgewählt sein können,
der Rest R in der jeweiligen Alkylenbrücke unabhängig voneinander für Wasserstoff, Hydroxy, einen geradkettigen oder verzweigtkettigen (C₁₋₈) Alkylrest, einen geradkettigen oder verzweigtkettigen (C₁₋₈) Alkoxyrest, -OC(O)R¹ oder -NHC(O)R¹ steht, wobei der Rest R¹ wie vorstehend definiert ist,
M* ein radioaktives Metall ist und
m und n jeweils eine ganze Zahl zwischen 1 und 5 ist.

Ein weiterer Gegenstand der vorliegenden Erfindung betrifft ein Verfahren zur Herstellung derartiger radioaktiver Metallkomplexe.

Als das radioaktive Metall M* können im Rahmen der vorliegendenErfindung insbesondere ein Nuklid des Kupfers, von seltenen Erden, Tc, In, Ga, Y oder Re eingesetzt werden. Vorzugsweise kann das radioaktive Metall M* aus ⁶⁴Cu oder ¹⁸⁸Re ausgewählt sein.

In einer bevorzugten Ausführungsform der vorliegenden Erfindung werden Metallkomplexe bereitgestellt, wobei in der Formel (I) A und B unabhängig voneineinander für H, -OR¹, -OC(O)R¹ oder -NHC(O)R¹ stehen oder A und B zusammen für =O stehen,
D jeweils eine Carboxylgruppe oder ein davon abgeleitetes Esterderivat ist,
X, Y und Z jeweils für ein unsubstituiertes oder substituiertes Pyridin stehen und der Rest R in der jeweiligen Alkylenbrücke unabhängig voneinander für Wasserstoff, Hydroxy, einen geradkettigen oder verzweigtkettigen (C₁₋₈) Alkylrest, einen geradkettigen oder verzweigtkettigen (C₁₋₈) Alkoxyrest, -OC(O)R¹ oder -NHC(O)R¹ steht, wobei der Rest R¹ wie vorstehend definiert ist.

In einer weiter bevorzugten Ausführungsform der vorliegenden Erfindung werden Metallkomplexe bereitgestellt, wobei in der Formel (I) A bzw. B für H bzw. OH stehen,
D jeweils eine -COOH Gruppe oder eine -COOMe Gruppe ist,
X, Y und Z jeweils für 2-Pyridinyl stehen und
der Rest R in der jeweiligen Alkyenbrücke jeweils für Wasserstoff steht,
und m und n jeweils 1 sind.

Insbesondere Bispidine mit zwei tertiären Amin- und vier Pyridindonoren an einem sehr starren, von Diazaadamantan abgeleiteten Gerüst gelten als effektive Liganden insbesondere für beispielsweise Kupfer, dessen resultierende Komplexe außergewöhnlich stabil sind.

Ein wichtiges Merkmal der erfindungsgemäßen Verbindungen ist, dass sie eine gegenüber den im Stand der Technik verfügbaren Komplexen verbesserte chemische und radiolytische Stabilität aufweisen. Außerdem bietet das Bispidin-Grundgerüst vielfältige Möglichkeiten zur Einführung von Biomolekülen.

Die vorliegenden Erfindung wird durch die nachstehenden, nicht-beschränkenden Beispiele weiter erläutert. Dabei werden repräsentativ die zwei speziellen, von Bispidin-9-ol und Bispidin-9-ol-dicarbonsäure abgeleiteten Chelatbildner verwendet.

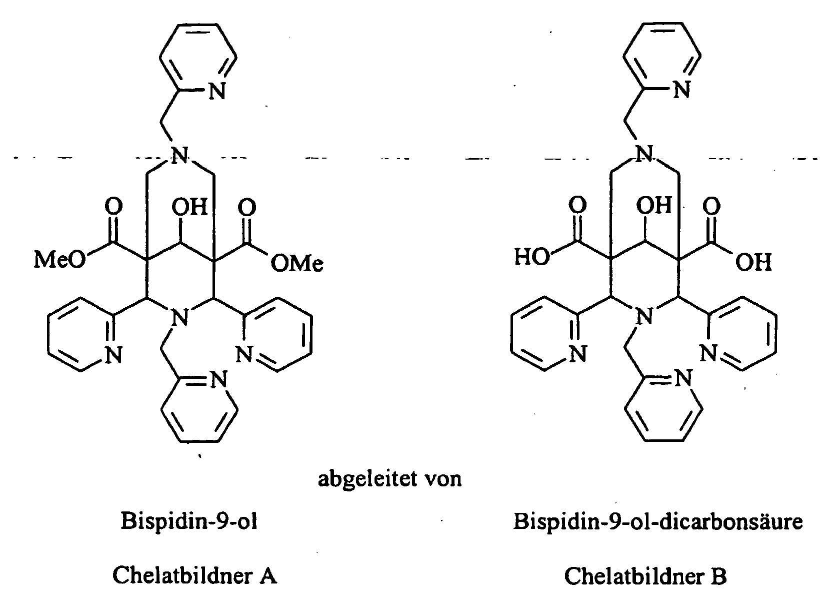

Der Chelatbildner A kann als (Dimethyl-9-hydroxy-2-{(1Z)1-[(1Z)-prop-2-en-1-ylidenamino]prop-1-en1-yl}-4-pyridin-2-yl-3,7-bis(pyridin-2-yl-methyl)-3,7-diazabicyclo[3.3.1]nonan-1,5-dicarboxylat bezeichnet werden. Der Chelatbildner B kann als (9-Hydroxy-2-{(1Z)-1-[(1Z)-prop-2-en-1-ylidenamino]prop-1-en1-yl}-4-pyridin-2-yl-3,7-bis(pyridin-2-yl-methyl)-3,7-diazabicyclo[3.3.1]nonan-1,5-dicarbonsäure bezeichnet werden.

Verbindungen der allgemeinen Formel (M* x Bisp) werden durch Zugabe einer wässrigen Lösung des radioaktiven Metalls (M*) zu einer wässrigen oder wässrig/organischen Lösung eines Bispidinderivates (Bisp) erhalten, wobei sich bei Raumtemperatur oder leichtem Erwärmen innerhalb weniger Minuten der Metallkomplex in über 95 %iger Ausbeute bildet.

### Beispiel 1: Chelatbildner A, abgeleitet von Bispidin-9-ol

500 mg des entsprechenden Bispidon-Derivats (0,844 mmol) werden in 8,3 ml Dioxan gelöst. Zu dieser Lösung gibt man 5,8 ml Wasser. Unter Eiskühlung und Rühren tropft man 0,216 g NaBH₄ (5,71 mmol), in 4,5 ml Wasser gelöst, zu. Die Reaktionslösung wird weitere 2 h unter Eiskühlung gerührt und für 2 Tage im Kühlschrank gelagert. Anschließend wird der pH-Wert der Lösung mit verd. HCl auf 1 eingestellt und das Reaktionsgemisch für 2-3 h bei Raumtemperatur gelagert. Nun engt man die Lösung um die Hälfte ein und bringt den pH-Wert mit 1 M NaOH auf 8. Im Anschluss wird 5 Mal mit CH₂Cl₂ extrahiert und die organische Phase eingeengt.
Ausbeute: 60 % d. Th., Fp: 167 °C.
Elementaranalyse (C₃₃H₃₃N₆O₅): ber. C: 66,77, H: 5,60, N: 13,48 %; gef. C: 64,84,
H: 6,03, N: 13,36 %
MS (ESI): 595 (M⁺)

### Beispiel 2: Cu(II)- Komplex mit Chelatbildner A

100 mg des Chelatbildners A (0,168 mmol) werden in 2 ml MeOH erhitzt. Dazu gibt man eine heiße Lösung von 31,5 mg Cu(NO₃)₂ (0,168 mmol) in 1 ml MeOH. Das Reaktionsgemisch wird abgekühlt und der Komplex mit Ether gefällt und abgetrennt.
Ausbeute: 21,9 % (d. Th.)
Elementaranalyse (C₃₃H₃₃N₈O₁₁Cu x H₂O): ber. C: 50,67, H: 4,38, N: 14,32 %;
gef. C: 49,53, H: 4,53, N: 14,00 %.
Dünnschichtchromatogram (RP-18, Aceton/ Ammoniumacetat (5 %ig) 1:1): R_{f} = 0,53
HPLC: (Jupiter Proteo 4 µ 90 A; Eluent A: CH₃CN + 0,1 % TFA, Eluent B: H₂O + 0,1 % TFA,
10%A->70%A (t= 20 min) : t_{R}= 10,76 min

### Beispiel 3: Herstellung eines ⁶⁴Cu-markierten Komplexes mit Chelatbildner A

Zu einer Lösung, die 0,1 mg Chelatbildner A in 100 µl 0,05M MES (Morpholinoethansulfonsäure) / naOH- Pufler mit einem pH-Wert von 5,3 enthält, werden 10 µl ⁶⁴CuCl₂ (0,1 M HCl) gegeben. Die Lösung wird 15 Minuten auf 37°C erwärmt.
Radiochemische Reinheit: > 95 %
Dünnschichtchromatogram (RP-18, Aceton/ Ammoniumacetat (5 %ig) 1:1): R_{f} = 0,53
HPL: (Jupiter Proteo 4 µ 90 A; Eluent A: CH₃CN + 0,1 % TFA, Eluent B: H₂O + 0.1 % TFA,
10 % A -> 70 % A (t= 20 min) : t_{R}= 10,76 min

### Beispiel 4: Chelatbildner B

190 mg Kalium-*tert*-butylat (1,69 mmol) werden im Kolben vorgelegt. Man gibt 100 mg Chelatbildner A (0,168 mmol) in 0,78 ml trockenem THF zu. Nach weiterer Zugabe von 1,5 ml THF wird die Lösung auf 0°C abgekühlt. Anschließend werden unter starkem Rühren 15 µl Wasser zugetropft. Nun wird die Lösung für 8 h am Rückfluss gehalten. Nach dem Abkühlen neutralisiert man mit 1 M HCl. Die Reaktionslösung wird zur Reinigung Ober eine RP-18 Kartusche gegeben.
Ausbeute: 25,5 % d. Th., Fp: 174 °C.
MS (ESI): 565 (M⁺)

### Beispiel 5: Cu(II)-Komplex mit Chelatbildner B

100 mg Chelatbildner B (0,179 mmol) werden in 2 ml MeOH erhitzt. Dazu gibt man eine heiße Lösung von 33,6 mg Cu(NO₃)₂ (0,179 mmol) in 1 ml MeOH. Das Reaktionsgemisch wird abgekühlt, der Komplex mit Ether gefällt und abgetrennt. Ausbeute: 19,3 % d. Th.
Dünnschichtchromatogram (RP-18, Aceton/ Ammoniumacetat (5 %ig) 1:1): R_{f} = 0,83
HPLC: (Jupiter Proteo 4 µ 90 A; Eluent A: CH₃CN + 0,1 % TFA, Eluent B: H₂O + 0,1 % TFA,
10%A->70%A (t= 20 min): t_{R}= 9,73 min

### Beispiel 6: Herstellung des ⁶⁴Cu-Komplexes mit Chelatbildner B

200 µl 10⁻⁴ M Ligandlösung (Chelatbildner B) - in MES (Morpholinoethansulfonsäure) / NaOH- Puffer mit einem pH-Wert von 5,4 - wurden mit 50 µl ⁶⁴CuCl₂ (0,1 M HCl) versetzt (-250 kBq) und 1 min bei Raumtemperatur geschüttelt.
Radiochemische Reinheit: > 99 %
Dünnschichtchromatogram (RP-18, Acetonitril + 0.1 % TFA/ Wasser + 0,1 % TFA 80:20) R_{f} = 0,16
HPLC: (Jupiter Proteo 4 µ 90 A; Eluent A: CH₃CN + 0,1 % TFA, Eluent B: H₂O + 0,1 % TFA,
10 % A -> 70 % A (t= 20 min): t_{R}= 9,40 min

### Beispiel 7: Stabilitätsuntersuchungen des ⁶⁷Cu-Komplexes mit Chelatbildner B im Rattenplasma

500 µg des Liganden B werdenm in 50 µl Wasser/CH₃CN gelöst und 50 µl ⁶⁷CuCl₂ (0.1M HCl) (-250 kBq) werden zugeben. Zu dieser Lösung gibt man 250 µl Phosphatpuffer (Sörensen, pH=7,4) und 250 µl Rattenplasma. Nun inkubiert man für 2 h bei 37°C. Anschließend wird kaltes EtOH (Volumen entspricht dem Gesamtvolumen der Lösung) zugesetzt. Man zentrifugiert 5 min bei höchster Stufe, nimmt die EtOH-Phase ab und überprüft den Gehalt an Aktivität (min. 100 kBq). Nun gibt man weitere 550 µl kalten EtOH hinzu und zentrifugiert erneut. Die EtOH-Lösung wird von den ausgefallenen Proteinen abgetrennt und eingeengt. Man gibt etwa 100 µl Wasser/CH₃CN-Lösung (9:1) hinzu und säuert mit 5 µl TFA an.
Dünnschichtchromatogram (RP-18, Acetonitril + 0,1 % TFA/ Wasser + 0,1 % TFA 80:20)
HPLC: (Jupiter Proteo 4 µ 90 A; Eluent A: CH₃CN + 0,1 % TFA, Eluent B: H₂O + 0,1 % TFA,
10%A->70%A(t=20min)

## Patentansprüche

1. Radioaktive Metallkomplexe auf Basis von Bispidin und dessen Derivate als Chelatbildner mit der folgenden allgemeinen Formel (I) 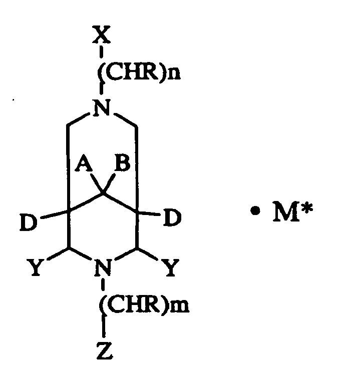 worin
A und B unabhängig voneinander für H, -OR¹, -SR¹, -NHR¹, -OC(O)R¹, -NHC(O)R¹ oder einen geradkettigen oder verzweigtkettigen (C₁₋₈) Alkylrest stehen oder A und B zusammen für =O oder =S stehen, wobei R¹ Wasserstoff, einen geradkettigen oder verzweigtkettigen (C₁₋₈) Alkylrest oder einen substituierten oder unsubstituierten (C₆₋₁₀) Arylrest darstellt,
D jeweils eine Carboxylgruppe oder ein davon abgeleitetes Derivat, ausgewählt aus Estern, Amiden und Peptiden, ist,
X, Y und Z unabhängig voneinander einen unsubstituierten oder substituierten Heterocyclus, ausgewählt aus Pyridin, Pyrimidin, Pyrazin, Thiophen, Furan, Pyrazol, Imidazol, Thiazol, Chinolin, Chinazolin und Chinoxalin, darstellen, wobei die Substituenten aus geradkettigen oder verzweigtkettigen (C₁₋₈) Alkylresten, Halogen, geradkettigen oder verzweigtkettigen (C₁₋₈) Alkoxyresten, Sulfonat, Carboxylat, Nitro, Cyan, Hydroxy, Benzyl oder Phenyl ausgewählt sein können,
der Rest R in der jeweiligen Alkylenbrücke unabhängig voneinander für Wasserstoff, Hydroxy, einen geradkettigen oder verzweigtkettigen (C₁₋₈) Alkylrest, einen geradkettigen oder verzweigtkettigen (C₁₋₈) Alkoxyrest, -OC(O)R¹ oder -NHC(O)R¹ steht, wobei der Rest R¹ wie vorstehend definiert ist,
M* ein radioaktives Metall ist und
m und n jeweils eine ganze Zahl zwischen 1 und 5 ist.

2. Radioaktive Metallkomplexe nach Anspruch 1, wobei in der Formel (I) A und B unabhängig voneineinander für H, -OR¹, -OC(O)R¹ oder -NHC(O)R¹ stehen oder A und B zusammen für =O stehen,
D jeweils eine Carboxylgruppe oder ein davon abgeleitetes Esterderivat ist,
X, Y und Z jeweils für ein unsubstituiertes oder substituiertes Pyridin stehen und
der Rest R in der jeweiligen Alkylenbrücke unabhängig voneinander für Wasserstoff, Hydroxy, einen geradkettigen oder verzweigtkettigen (C₁₋₈) Alkylrest, einen geradkettigen oder verzweigtkettigen (C₁₋₈) Alkoxyrest, -OC(O)R¹ oder -NHC(O)R¹ steht, wobei der Rest R¹ wie vorstehend definiert ist.

3. Radioaktive Metallkomplexe nach Anspruch 1 oder 2, wobei in der Formel (I) A bzw. B für H bzw, OH stehen,
D jeweils eine -COOH Gruppe oder eine -COOMe Gruppe ist,
X, Y und Z jeweils für 2-Pyridinyl stehen und
der Rest R in der jeweiligen Alkyenbrücke jeweils für Wasserstoff steht,
und m und n jeweils 1 sind.

4. Radioaktive Metallkomplexe nach Anspruch 1, 2 oder 3, wobei das radioaktive Metall M* ein Nuklid des Kupfers, von seltenen Erden, Tc, In, Ga, Y oder Re ist.

5. Radioaktive Metallkomplexe nach einem der vorhergehenden Ansprüche 1 bis 4, wobei das radioaktive Metall M* ⁶⁴Cu oder ¹⁸⁸Re ist.

6. Radioaktive Metallkomplexe nach einem der vorhergehenden Ansprüche 1 bis 5 zur Verwendung in der nuklearmedizinischen Diagnostik.

7. Radioaktive Metallkomplexe nach einem der vorhergehenden Ansprüche 1 bis 5 zur Verwendung in der internen Radionuklidtherapie.

8. Verfahren zur Herstellung der radioaktiven Metallkomplexe auf Basis von Bispidin und dessen Derivate als Chelatbildner nach einem der vorhergehenden Ansprüche 1 bis 5, worin ein radioaktives Metall mit einem Bispidinderivat in einer wässrigen oder wässrig-organischen Lösung gemischt und auf eine Temperatur zwischen 20 und 50 °C gebracht und bei dieser Temperatur eine Stunde gehalten wird.

## Claims

1. A radioactive metal complex based on bispidine and the derivatives thereof as chelating agent having the following general formula (I) 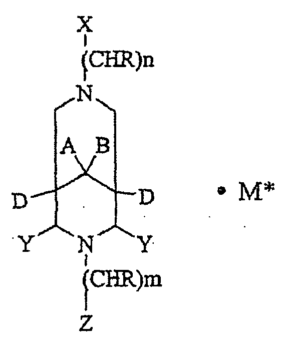 in which
A and B are independently of one another H, -OR¹, -SR¹, -NHR¹, -OC(O)R¹, -NHC(O)R¹ or a straight-chain or branched-chain (C₁₋₈) alkyl radical, or A and B together are =0 or =S, where R¹ is hydrogen, a straight-chain or branched-chain (C₁₋₈) alkyl radical, a substituted or unsubstituted (C₆₋₁₀) aryl radical,
D is in each case a carboxyl group or a derivative derived therefrom and selected from esters, amides and peptides,
X, Y and Z are independently of one another an unsubstituted or substituted heterocycle selected from pyridine, pyrimidine, pyrazine, thiophene, furan, pyrazole, imidazole, thiazole, quinoline, quinazoline and quinoxaline, where the substituents may have been selected from straight-chain or branched-chain (C₁₋₈) alkyl radicals, halogen, straight-chain or branched-chain (C₁₋₈) alkoxy radicals, sulfonate, carboxylate, nitro, cyano, hydroxy, benzyl or phenyl,
the radical R in the respective alkylene bridge is independently of one another hydrogen, hydroxy, a straight-chain or branched-chain (C₁₋₈) alkyl radical, a straight-chain or branched-chain (C₁₋₈) alkoxy radical, -OC (O) R¹ or -NHC (O) R¹, where the radical R¹ is as defined above,
M* is a radioactive metal, and
m and n is in each case an integer between 1 and 5.

2. The radioactive metal complex as claimed in claim 1, where, in formula (I), A and B are independently of one another H, -OR¹, -OC(O)R¹ or -NHC(O)R¹, or A and B are together =O,
D is in each case a carboxyl group or an ester derivative derived therefrom,
X, Y and Z are in each case an unsubstituted or substituted pyridine, and
the radical R in the respective alkylene bridge is independently of one another hydrogen, hydroxy, a straight-chain or branched-chain (C₁₋₈) alkyl radical, a straight-chain or branched-chain (C₁₋₈) alkoxy radical, -OC(O)R¹ or -NHC(O)R¹, where the radical R¹ is as defined above.

3. The radioactive metal complex as claimed in claim 1 or 2, where, in formula (I), A and B are respectively H and OH,
D is in each case a -COOH group or a -COOMe group, X, Y and Z are in each case 2-pyridinyl, and
the radical R in the respective alkylene bridge is in each case hydrogen,
and m and n are each 1.

4. The radioactive metal complex as claimed in claim 1, 2 or 3, where the radioactive metal M* is a nuclide of copper, of rare earths, Tc, In, Ga, Y or Re.

5. The radioactive metal complex as claimed in any of the preceding claims 1 to 4, where the radioactive metal M* is ⁶⁴Cu or ¹⁸⁸Re.

6. The use of the metal complexes as claimed in any of the preceding claims 1 to 5 in nuclear medical diagnosis.

7. The use of the metal complexes as claimed in any of the preceding claims 1 to 5 in internal radio nuclide therapy.

8. A method for preparing the radioactive metal complexes based on bispidine and the derivatives thereof as chelating agents as claimed in any of the preceding claims 1 to 5, in which a radioactive metal is mixed with a bispidine derivative in an aqueous or aqueous-organic solution and brought to a temperature between 20 and 50°C and kept at this temperature for one hour.

## Revendications

1. Complexes métalliques radioactifs à base de bispidine et de ses dérivés utilisés en tant qu'agent chélateur avec la formule générale suivante (I) 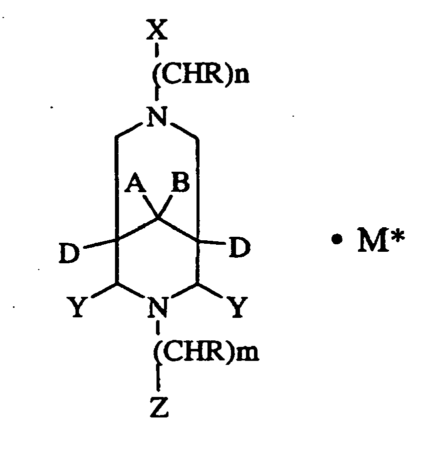 où
A et B sont indépendants l'un de l'autre pour H, -OR1,-SR-1,-NHR1, -OC(0)R1, -- NHC(0)R1 ou un radical alcoyle en chaîne droite ou ramifiée (C1-8) ou bien A et B sont ensemble pour =0 ou =S, où R 'hydrogène' constitue un radical alcoyle en chaîne droite ou ramifiée (C1-8) ou un radical alcoyle substitué ou non-substitué (C6-10),
D est respectivement un groupe carboxyle ou un de ses dérivés, sélectionné parmi les esters, les amides et les peptides,
X, Y et Z constituent indépendamment l'un de l'autre un hétérocyle non-substitué ou substitué, sélectionné parmi la pyridine, la pyrimidine, la pyrazine, le thiophène, le furanne, le pyrazole, l'imidazole, le thiazole, la quinoléine, la quinazoline et la quinoxaline, où les substituants d'un radical alcoyle en chaîne droite ou ramifiée (C1-8) l'halogène, d'un radical alcoxy en chaîne droite ou ramifiée C1-8), le sulfonate, le carboxylate, le nitre, le cyan, l'hydroxy, le benzyle ou le phényl, peuvent être sélectionnés, Le radical R est dans les ponts alkyl respectivement indépendants l'un de l'autre pour l'hydrogène, l'hydroxy, un radical alcoxy en chaîne droite ou ramifiée (C1-8), un radical alcoxy OC (0)R1 ou -NHC(O)R1 en chaîne droite ou ramifiée C1-8), où le radical R1 est défini comme ci-dessus,
M * est un métal radioactif et
m et n respectivement un nombre entier compris entre 1 et 5.

2. Complexes métalliques radioactifs selon la revendication 1, où dans la formule (I) A et B sont indépendants l'un de l'autre pour H,-OR1,-OC(0)R1 ou -NHC(0)R1 ou A et B sont ensemble pour =0,
D est respectivement un groupe carboxyle ou un dérivé d'ester, X, Y et Z sont respectivement pour la pyridine non-substituée ou substituée et
le radical R est respectivement dans des ponts alkyl indépendants l'un de l'autre pour l'hydrogène, l'hydroxy, un radical alkyl en chaîne droite ou ramifiée (C1-8), un radical alkoxy en chaîne droite ou ramifiée -OC(0)R1 ou -NHC(0)R1, où le radical R1 est défini comme ci-dessus.

3. Complexes métalliques radioactifs selon la revendication 1 ou 2, où dans la formule (I) A ou B sont pour H ou OH,
D est respectivement un groupe-COOH ou un groupe COOMe,
X, Y et Z, sont respectivement pour pyridinyl-2 et
le radical R 25 est respectivement dans les ponts alkyl pour l'hydrogène,
et m et n sont respectivement 1.

4. Complexes métalliques radioactifs selon la revendication 1, 2 ou 3, où le métal radioactif M * est un nucléide de cuivre, de terres rares, Tc, In, Ga, Y ou Re.

5. Complexes métalliques radioactifs selon les revendications précédentes 1 à 4, où le métal radioactif est M* 64CU ou 188Re.

6. Complexes métalliques radioactifs selon l'une des revendications précédentes 1 à 5 pour l'utilisation à des fins de diagnostic dans la médecine nucléaire.

7. Complexes métalliques radioactifs selon l'une des revendications précédentes 1 à 5 pour l'utilisation dans la thérapie radionucléide.

8. Procédés de fabrication de composés métalliques radioactifs à base de bispidine et de ses dérivés en tant qu'agent chélateur selon l'une des revendications 1 à 5, où un métal radioactif est mélangé avec un dérivé de bispidine dans une solution aqueuse ou aqueuse organique et porté à une température entre 20 et 50 ° C, et est maintenue une heure à cette température.
